# EUROPEAN PATENT APPLICATION

(11) **EP 4 368 027 A1**
(43) Date of publication of application: **15.05.2024**
(21) Application number: 22383070.4
(22) Date of filing: 08.11.2022
(51) Int. Cl.: A23J 1/00, A23J 3/20, C12N 1/16

(54) **PROCESS FOR THE MANUFACTURE OF FOOD PRODUCTS WITH CRABTREE-NEGATIVE YEASTS**

(71) Applicant: Moa Biotech, S.L., 31110 Noain, Pamplona (ES)
(72) Inventor: SÁNCHEZ GÓMEZ, Susana, 31110 Noain, Pamplona (ES); EMPARANZA GARCÍA, Bosco, 31110 Noain, Pamplona (ES); PERTIERRA RODRIGUEZ, Miguel, 31110 Noain, Pamplona (ES); SAFONT RESARDI, Bárbara, 31110 Noain, Pamplona (ES); CASTANDER ILLARRAMENDI, Kizkitza, 31110 Noain, Pamplona (ES); CAVERO ASIÁIN, Maria Eugenia, 31110 Noain, Pamplona (ES)
(74) Representative: Hoffmann Eitle

(57) **Abstract**

The present invention refers to a process for obtaining edible biomass departing from byproducts of the food industry and fermenting the biomass in the presence of a Crabtree-negative yeast; to the edible biomass thus obtained; to the use of the edible biomass for the manufacture a food product, or to a food product comprising the edible biomass obtained by means of the above cited process.

## Description

### FIELD OF THE INVENTION

The present invention refers to the food science or food technology field. Particularly, the present invention refers to a process for obtaining edible biomass departing from by-products of the food industry; to the edible biomass thus obtained; to the use of the edible biomass for the manufacture a food product: or to a food product comprising the edible biomass obtained by means of the above cited process.

### STATE OF THE ART

The projections show that feeding a world population of almost 10 billion people in 2050 would require raising overall food production by 70 percent. Currently, this food gap is being related to unsustainable agri-food practices. At the same time, we urgently need to cut greenhouse gas (GHG) emissions from agricultural production and stop conversion of remaining forests to agricultural land. Thus, there is an enormous interest in developing new sources of food, sustainable, nutritional and healthy.

Although other non-animal alternatives are marketed, they present some drawbacks as the organoleptic properties, deforestation (soy) and low amount of some amino acids needed in our diet (i.e., lysine).

In this context, the current plant-based alternatives to meat, dairy and eggs face a number of formulations challenges, regarding the organoleptic and functional properties that can mimic the animal-based proteins and therefore substitute them. The market trends lye in 3 main areas: proteins from plants, fermentation and animal culture.

Use of microorganisms as an alternative source of SCP (Single Cell Proteins) arises with great potential due to all the advantages they present, such as their independence from seasonal or climatic factors and the use of facilities in very small areas, which reduces deforestation caused by other alternatives (vegetable and / or animal). In addition, the water consumed, and the CO₂ emitted into the atmosphere are reduced compared to animal protein (approximately 98% and 85% respectively). Besides this, they presented a high nutritional profile, with all the amino acids recommended by FAO and the presence of other compounds such as vitamins, omega fatty acids and beta-glucans that are related with an immunomodulatory role.

By 2050, the world would need to produce 1,250 million tonnes of meat and dairy per year to meet global demand for animal-derived protein at current consumption levels. However, growing demand for protein will not be met sustainably by increasing meat and dairy production because of the low efficiency of converting feed to meat and dairy products. New solutions are needed. Single cell protein (SCP), i.e., protein produced in microbial and algal cells, is an option with potential. Much of the recent interest in SCP has focused on the valorisation of side streams by using microorganisms to improve their protein content, which can then be used in animal feed.

Protein can also be provided through the cultivation of various microbes and algae, preferably those which contain more than 30% protein in their biomass, and which can provide a healthy balance of essential amino acids. Current marketed SCP are based on mycoprotein, based on fungal cells, that are limited to meat analogues and there are a concern regarding the presence of mycotoxins.

Due to the climate change and the diversity of by-product around the world, similar ingredients with high nutritional profile are difficult to find in different regions and the scarcity of some of them are a real concern.

Beside this, there is need and consumer tendence for clean label products and non-animal derived ingredients and adjuvants. Those ingredients display some technological properties such as gelling, emulsifying and foaming properties to be added to dairy and meat analogues and thus, substituting animal proteins such as casein or albumin. Currently modified vegetables protein are used (although this approach did not solve the environment impact) or synthetic polymers as methylcellulose (non-natural ingredient).

The present invention is focused on solving this problem and an innovative method for obtaining edible biomass departing from by-products of the food industry is herein described. Such as it is indicated above, this suggested method uses by-products of the food industry, so it is helping to solve an important problem by producing edible biomass without using limited biological and physical resources of the planet. Moreover, according to the method herein proposed, a single edible biomass final product, with uniform quantitative and qualitative components, may be obtained, departing from a variety of by-products of the food industry, thus streamlining, facilitating, and optimising the industrial process of biomass production. So, it is possible to use a wide range of agri-food by-products such as those derived from sugar, potatoes, cereals, vegetables among others. This fact results in a sustainable process with minimal environmental impact. The obtention of similar ingredient from different by-products would allow to produce them locally and therefore, the food chain would not depend on imports from other countries, decreasing the risk of ingredient shortages due to climate change or economic, political and social issues.

### DESCRIPTION OF THE INVENTION

### Brief description of the invention

The present invention refers to a process for obtaining edible biomass (wherein said biomass product comprises for instance one or more of single cell protein, cell lysate, protein concentrate, protein isolate, biomass extract and biomass hydrolysate) departing from by-products of the food or feed industry; to the edible biomass thus obtained; to the use of the edible biomass for the manufacture a food product: or to a food product comprising the edible biomass obtained by means of the above cited process.

The method of the invention is based on the fermentation of by-product solution or suspension (for example solubilized carbon, nitrogen or other micronutrients) in the presence of specific microorganisms, particularly Crabtree-negative yeasts. By using by-products of the food industry, the inventors of the present invention are contributing to solve an important problem because they are able to produce edible biomass taking advance of by-products, and consequently without using limited biological and physical resources of the planet. Moreover, according to the method herein proposed, a single edible biomass final product, with uniform quantitative and qualitative components, may be obtained, departing from a variety of by-products of the food industry, thus streamlining, facilitating, and optimising the industrial process of biomass production.

So, the first embodiment of the present invention refers to a process for obtaining edible biomass (hereinafter *process of the invention)* which comprises: a) Placing by-products of the food industry in suitable conditions for their solubilization and/or resuspension, b) Fermentation of the by-product solution or suspension in the presence of a Crabtree-negative yeast, and c) inactivation of the Crabtree-negative yeast to obtain the edible biomass.

The by-products are solubilized or resuspended in aqueous solutions at different pH depending on their origin (pH=0,5-14), with or without the presence of enzymes and in combination with physical (high temperature or sonication) or mechanical (high shear homogenization) treatment. If needed the media can be supplemented with other macro and micronutrients for the yeast growth.

The harvest biomass is inactivated by thermal or not thermal treatments. Additionally, the biomass can be lyses, for instance by autolysis, enzymatic, homogenization, high pressure, ultrasounds or other lysis procedures. In order to obtain functional ingredient with specific technological properties as emulsifying and gelling properties, different methods have been described with the objective of the extraction of certain components of the yeast cell.

In a preferred embodiment, the process of the invention comprises the use of Crabtree-negative yeast is selected from: *Candida utilis, Kluyveromyces marxianus, Debaryomyces hansenii* or *Yarrowia lipolytica.* Other Crabtree negative yeasts which may be used are selected from: *Candida cylindrace, Saccharomyces cabtree negative species, Hansenula spp., Pichia spp. Kluyveromyces spp, Brettanomyces spp., Kamogataella spp, Ogatea angusta, Schizosaccharomyces pombe, Lindnera jardinii,* or *Wickerhamomyces anomalus* among others.

So, this first embodiment also refers to the use of a Crabtree-negative yeast for obtaining edible biomass departing from by-products of the food industry, preferably to the use of Crabtree-negative yeast is selected from: *Candida utilis, Kluyveromyces marxianus, Debaryomyces hansenii* or *Yarrowia lipolytica.* Other Crabtree negative yeasts which may be used are selected from: *Candida cylindrace, Saccharomyces cabtree negative species, Hansenula spp., Pichia spp. Kluyveromyces spp, Brettanomyces spp., Kamogataella spp, Ogatea angusta, Schizosaccharomyces pombe, Lindnera jardinii,* or *Wickerhamomyces anomalus* among others.

In a preferred embodiment of the invention, the process of the invention is characterized in that a single edible biomass final product, with uniform quantitative and qualitative components, is obtained, departing from a variety of by-products of the food industry.

In a preferred embodiment of the invention, the edible biomass comprises at least 30% of protein content, at least 10% of soluble fibre and/or at least 1% of fat content.

In a preferred embodiment of the invention, the edible biomass final product comprises: 30 to 80% of protein content, 10 to 70% of soluble fibre and/or 1 to 60% of fat content.

In a preferred embodiment of the invention, the by-products are non-animal derived by-products.

In a preferred embodiment of the invention, the by-products are vegetal derived by-products.

The second embodiment of the present invention refers to a process for obtaining a food product which comprises: a) Obtaining edible biomass by following the above defined *process of the invention,* and b) fractionating to obtain the food product.

In a preferred embodiment the fractionation is carried out by inactivation and/or lysis. After the lysis, the treatment of different cellular fractions leads to a final ingredient with similar nutritional profile and different technological properties. This procedure allows to obtain several ingredients to be included in a wide range of applications. Particularly, the first step includes the inactivation and /or lysis of the yeast. This procedure includes autolysis, chemical lysis (solvents, detergents, osmotic shock), physical disruption (thermal treatment-including autoclave-, sonication, freeze-thaw, hot water or steam, ultrasounds), mechanical lysis (high pressure, homogenization) or enzymatic treatment. After that, the samples can be fractionated directly or after a centrifugation step (cell wall). The fractionating steps include alkaline, acid or neutral treatment, buffer citrate treatment or enzymatic treatment at different incubation times, combined or not with other physical o mechanical treatment. An optional third step that include separation can be done by the performance of at least one of the following procedures: physical separation (filtration or centrifugation), acidic precipitation and organic solvent precipitation. Finally, the final ingredients can be dried. Those ingredients were characterized by their nutritional profile and technological properties regarding gelling, foaming, emulsifying activity as well as solubility, water absorption and oil absorption capacity. With those processes no specific purifications steps were used, but enrichment or availability enhancement of different fractions that lead to several functional ingredients. Minimal modifications of the downstream process let the obtention of ingredients with similar nutritional profile to the initial biomass and with a wide range of functional properties. Depending on the combination of the 3 steps (lysis, fractioning and separation), the final ingredient displays different technological properties.

The third embodiment of the present invention refers to edible biomass obtained by the *process of the invention,* preferably comprising: at least 30% of protein content, at least 10% of soluble fibre and/or at least 1% of fat content; more preferably 30 to 80% of protein content, 10 to 70% of soluble fibre and/or 3 to 60% of fat content.

The fourth embodiment of the present invention refers to the use of the above defined edible biomass to the manufacture of a food product.

The fifth embodiment of the present invention refers to a food product obtainable by means of the process described in the second embodiment of the present invention.

The sixth embodiment of the present invention refers to a food product comprising the above defined edible biomass.

In a preferred embodiment, the food product of the invention comprises: at least 30% of protein content, at least 10% of soluble fibre and/or at least 1% of fat content; more preferably 30 to 80% of protein content, 10 to 70% of soluble fibre and/or 3 to 60% of fat content.

For the purpose of the present invention the following terms are defined:
- The term "comprising" means including, but not limited to, whatever follows the word "comprising". Thus, use of the term "comprising" indicates that the listed elements are required or mandatory, but that other elements are optional and may or may not be present.
- The term "consisting of" means including, and limited to, whatever follows the phrase "consisting of'. Thus, the phrase "consisting of" indicates that the listed elements are required or mandatory, and that no other elements may be present.

### Description of the figures

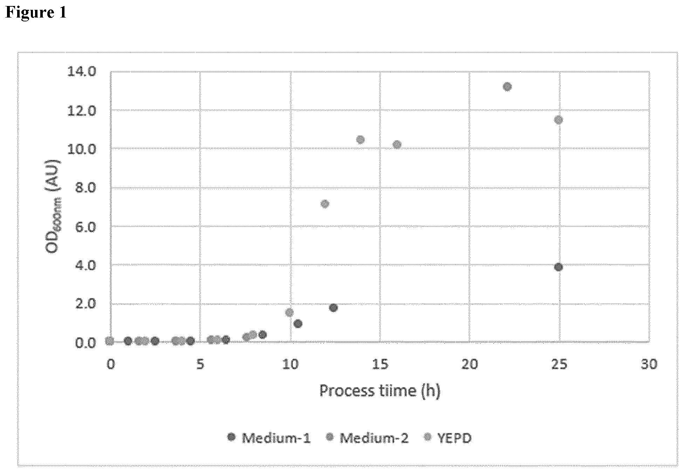

**Figure 1****.** *Kluyveromyces marxianus* growth in different by-product media (medium-1 and medium-2) and in a rich media (YEPD).

### Detailed description of the invention

The present invention is illustrated by means of the Examples set below without the intention of limiting its scope of protection.

### Example 1. By-product treatment

### Media preparation

By- products were weighted and resuspended in different aqueous solution (neutral, basic or acid) in combination with temperature treatments in order to solubilize most of the components (room temperature or 80°C). After that, the soluble components were neutralized and used directly for media preparation. Avoiding the removal of the acid and base and the dried steps, the process is more scalable and accessible for industry. In **Table 1,** a selection of some by-products which have been treated in the present invention are shown. Representants of each group of by-products were selected: sugar-rich by-product (derived from sugar industry, containing mono and disaccharides), carbohydrate rich by-product, fiber-rich by-product and lipid or protein-rich by-products. If needed, other macro and micronutrient are added to the media. The media was sterilized by autoclaving or heating the media up to 100°C. When it is required, some nutrients were sterilized by filtration.

**Table 1. By-product used and their nutritional profile**

| **By-products** | **Moisture (%)** | **Sugars (%)** | **Carbohydrates (%)** | **Fat (%)** | **Proteins (%)** | **Fiber (%)** |
|---|---|---|---|---|---|---|
| Bread Crusts | 20 | 5 | 46 | 5 | 9 | 5 |
| Wheat Flour | 13 | 3 | 28 | 5 | 16 | 26 |
| Wheat Bran | 10 | 0 | 24 | 5 | 17 | 37 |
| Regrinded Pasta | 10 | 3 | 55 | 2 | 14 | 2 |
| Sunflower Flour | 9 | 0 | 0 | 0 | 29 | 48 |
| Highly Oleic Sunflower | 9 | 0 | 0 | 0 | 30 | 47 |
| Grape Seeds | 13 | 0 | 0 | 0 | 9 | 75 |
| Canola Flour | 8 | 0 | 0 | 0 | 35 | 35 |
| Beetroot Molasses | 28 | 46 | 0 | 0 | 0 | 0 |
| Molasses derivate 1 | 28 | 57 | 0 | 0 | 0 | 0 |
| Molasses derivate 2 | 28 | 57 | 0 | 0 | 0 | 0 |
| Molasses derivate 3 | 31 | 67 | 0 | 0 | 0 | 0 |
| Okara | 76 | 1 | 0 | 2 | 7 | 13 |
| Corn Flour | 14 | 9 | 54 | 0 | 10 | 9 |
| Used cooking oil | 0 | 0 | 1 | 99 | 0 | 0 |
| Ranges | 0-76 | 0-67 | 0-55 | 0-99 | 0-35 | 0-75 |

The by-products derived from sugar industry were directly diluted in water. The other by-products were subjected to other treatments. The most promising treatments where those that include high temperature treatment combined with alkali o acid treatment (preferably acid) as shown in **Table 2.** Those treatments were able to solubilize more that 80% of the by-product.

**Table 2. By-products treated and composition**

| **Component (%)** | **Bread Crusts before treatment (theoretically)** | **Bread Crusts-1** | **Bread Crusts-2** | **Bread Crusts-3** | **Bread Crusts-4** |
|---|---|---|---|---|---|
| Glucose | 0.07-0.1 | 0.19 | 1.37 | 0.14 | 1.48 |
| Fructose | 0.13 | 0.25 | 0.34 | 0.18 | 0.38 |
| Sucrose | 0.25 | 0.19 | <0.10 | 0.24 | <0.10 |
| Maltose | 0.17 | 0.16 | 0.66 | 0.13 | 0.71 |
| Starch | 4.1 | 2.4 | 2.1 | 4 | 2.1 |
| Humidity | 90 | 91.4 | 89.3 | 90.4 | 88.6 |

| | | | | | |
|---|---|---|---|---|---|
| *Treatments: 1: HCl room temperature, 2: HCl* + *80°C, 30 min; 3: HCl room temperature and autoclaved and 4: HCl* + *80°C, 30 min+ autoclave.* | | | | | |

With those treatments, more than 80% of the by-products were solubilized and also the carbon source (starch, mono and disaccharides). With some treatments, a partial saccharification of the starch were obtained.

After the treatment, other macronutrients and micronutrients were added to the by-product during the conditioning. Those compounds include other by-products, inorganic nitrogen source, organic nitrogen sources, MgSO₄, phosphate salts, trace salts or other growth factors needed (such as vitamins).

The media was sterilized by autoclaving or heating the media up to 100°C. When it is required, some nutrients were sterilized by filtration.

### Example 2. Crabtree negative yeasts growth in by-products

Several yeasts, for instance *Kluyveromyces marxianus, Candida utilis, Yarrowia lipolytica* and *Debaryomyces hansenni,* were used for the evaluation of the feasibility of the by-products-based growth media. Several strains were used and one of them was selected for further optimization. The growth rate was monitored by the OD of the culture and, similar or even higher growth rate was obtained with a medium based on a by-product (medium-2) in comparison with a commercial rich media (YEPD) as **Figure 1** shows.

The increase of carbon source leads to an increase in the biomass concentration as describe for Crabtree negative yeast **(Table 3).** The efficiency of the process leads to conversion rates higher than 70% **(Table 4).**

**Table 3. Yarrowia lipolytica's biomass after the growth in different media**

| **Sugar by-product 2** | **Biomass (g/L)** |
|---|---|
| 40 g/L | 12.0 |
| 50 g/L | 15.3 |
| 60 g/L | 18.0 |
| 70 g/L | 19.7 |

**Table 4. Conversion rate of Debaryomyces hansenii's biomass from dried bread crust4**

| Medium | **By-product C** | **Biomass (g/L)** | **Conversion rate** |
|---|---|---|---|
| **Medium-1** | 5 g/L | 4.6 g/L | 99.2 |
| **Medium-2** | 8 g/L | 7.30 g/L | 91.28 |
| **Medium-3** | 15 g/L | 13.5 | 73.1 |
| **Medium-5** | 20 g/L | 15.7 | 81.25 |
| **Medium-5** | 30g/L | 23.3 | 78.7 |
| **YEPD ¹** | n/a ² | 17.96 | n/a |

| | | | |
|---|---|---|---|
| ¹ *Rich media (Yeast extract peptone dextrose); ² not applicable* | | | |

### Example 3. Same by-product, different microorganisms and similar ingredient

Different microorganisms *(Kluyveromyces marxianus* and *Candida utilis)* were grown in the same by-product (sugar derived by-product). The fermentation process includes several steps: Preinoculum in a rich media or in the by-product media, for 8-24h. The culture temperature ranges 20-45°C (depend on the strain).

The fermentation process consists of a batch or fed-batch fermentation (depending on the microorganism) with a duration between 24-72h. The batch phase lasted until the carbon source is depleted and fed-batch begins. Modification of specific fermentation condition led to different ingredients.

The by-product was diluted and supplemented with a mixture of organic and inorganic nitrogen source (yeast extract and (NH₄)₂SO₄), phosphate salts and Mg₂SO₄. The media was sterilized by autoclaved in a bioreactor. An overnight culture of the crabtree negative yeasts were added to each media and the culture was incubated for 24-48h at 35°C under agitation and aireation.

After that, the biomass was harvest, inactivated by heat and dried by spray drier. The biomass derived from different microorganism were characterized according to their nutritional profile as **Table 5** shows.

The modulation of the fermentation strategy allows us to set up the composition of certain compounds in order to obtain similar ingredients from different by-products **(Table 5)**

**Table 5. Nutritional profile of two different biomass derived from two crabtree negative yeast**

| **Parameter (g/100g)** | ***Kluyveromyces marxianus* ¹** | ***Candida utilis*** |
|---|---|---|
| Moisture | 3.8 | 3.7 |
| Fat | 4.1 | 9.9 |
| Protein | 38.2 | 40.8 |
| Ash | 8.6 | 7.6 |
| Total carbohydrate | 12.6 | 7.0 |
| Sugar | 0.9 | 2.0 |
| Dietary fiber | 31.9 | 31.0 |

| | | |
|---|---|---|
| *¹Media of 8 batches* | | |

### Example 4. Different by-product, same microorganism and similar ingredient

Different by-products were used for the growth of one microorganism: *Debaryomyces hansenii.* Both by products present different macronutrients (**Table 6**) with the main differences in carbohydrates and fiber content.

**Table 6. Nutritional profile of by-products tested**

| **Parameter (g/100g)** | **Dried Bread Crusts** | **Wheat Bran** |
|---|---|---|
| Moisture | 10 | 10 |
| Carbohydrates | 75 | 24 |
| Proteins | 10.9 | 17 |
| Total Fat | 6.7 | 5 |
| Total Dietary Fiber | 5.8 | 37 |
| Salt/Ash | 1.8 | 6 |

Both by-products were treated with an alkali solution at 80°C, neutralized and autoclaved, obtaining a solubility rate of >80%. Salts and other micronutrients were added. An overnight culture of the crabtree negative yeast were added to each media and the culture was incubated for 24-48h at 25°C under agitation in baffled flasks. After that, the biomass was recovered, inactivated by heat and air dried. The composition of the biomass obtained with both bioprocesses are summarized in **Table 7.**

**Table 7. D. hansenni biomass obtained from 2 different by-products**

| **Parameter (g/100g)** | ***D. hansenii* 1** | ***D. hansenii* 2** |
|---|---|---|
| Moisture | 8.02 | 7.25 |
| Fat | 2.37 | 3.12 |
| Protein | 43.93 | 45.01 |
| Ash | 9.98 | 8.95 |
| Total carbohydrate | 5.30 | 5.6 |
| Sugar | nd | nd |
| Dietary fiber | 31.4 | 29.6 |

| | | |
|---|---|---|
| *Nd: not done* | | |

### Example 5. Different by-products, different microorganism and similar ingredient

Another microorganism, *Yarrowia lipolytica,* was used for another sugar by-product valorization. The by-product-based media were prepared as follows. Firstly, the by-product was solubilized, autoclaves and other pre-sterilized compounds were added (MgSO₄ · 7 H₂O, (NH₄)₂SO₄ and/or yeast extract and phosphate salts. The incubation temperature was 25 +/- 1 °C and the agitation rate was 500-800 rpm. The pH was controlled with 2.5M H₃PO₄ as acid and 25% KOH as base. Aeration rate was stablished at 1 vvm. After 48 h, the biomass was harvest by centrifugation and freeze-dried. As **Table 8** shows, the nutritional composition of among the biomass were similar.

**Table 8. Nutritional profile of the biomass derived from different microorganism and by-products**

| **Parameter (g/100g)** | ***Y. lipolytica* 1** | ***Y. lipolytica* 2** | ***D. hansenii* 1** |
|---|---|---|---|
| Moisture | 13.38 | 1.89 | 8.02 |
| Fat | 4.68 | 5.54 | 2.37 |
| Protein | 47.41 | 49.99 | 43.93 |
| Ash | 4.05 | 8.27 | 9.98 |
| Total carbohydrate | 8.58 | 3.71 | 5.30 |
| Sugar | 3.4 | 3.6 | nd |
| Dietary fiber | 21.9 | 30.6 | 31.4 |

### Example 6. Food application: Sauces

*Kluyveromyces* derived edible biomass presents a good solubility, emulsifying properties and partial water and oil retention capacity. Several food applications have been done with this ingredient substituting animal derived ingredients and giving additional value to the final product. One example is pesto sauce, in which the parmigiano cheese was substituted with this edible biomass and provide additional beneficial claims as source of protein, vegan, source of vitamin B1 and B2 and high in vitamin B8 and B9. The process of making the sauce consist of grinding the nuts, spices, vinegar and flavours in a cutter until all is well missed. And the last step is to create and emulsion by adding the oil to the cutter.

### Example 7. Food application: Pasta

Same biomass (9,73%) was included in pasta, substituting egg and bringing to the product additional claims as source of protein, source of fiber, vegan, source of vitamin B6 and high in vitamins B1, B2, B8 and B9. The process of making the fresh pasta consist of creating a dough mixing water, flower or semolina, salt and MOA protein. Once the dough has rested for 1h the dough is formed to create the desired shape

### Example 8. Food application: Cheese analogues

Same biomass (16%) was used to prepared cheese analogues and bringing the product relevant claims as source of protein, source of fiber, vegan, source of vitamin B6, high in vitamin B1, B2, B8 and B8, as well as high in phosphorous and iron. The process of making cheese analogues consist of grinding the nuts and mixing it with culture starter, salt and moa protein. Once the shape of the cheese is made, it will be placed at room temperature for 4-7 days. Once the fermentation period is finished the cheese will be covered by spices and salt.

### Example 9. Fractioning for gelling, emulsifying and foaming properties (prototype KS22AcEt)

Yeast cells were lysed by physicochemical treatment using a mild alkaline (pH from 8 to 11) sodium hexametaphosphate buffer and heating the suspension to 65°C for 2h. After that, cell wall was removed and resuspended in buffer citrate for its treatment at 121°C for 20'. After these treatments, two consecutive precipitation steps were carried out, neutralizing the solution to pH = 4,5 to 5,5 and adding absolute ethanol. After that, the sample was dried, and technological properties achieved were gelling activity in hot conditions, emulsifying and foaming activities, while nutritional properties weren't significantly changed.

### Example 10. Fractioning for gelling and emulsifying properties (prototype KD12F)

Yeast cells were lysed by mechanical disruption by pressure. After that, the cell wall was removed and resuspended in buffer citrate and treated for 20' at a temperature that range from 80°C-121°C . After that, the sample was directly dried. The nutritional profiles obtained were similar and the technological properties achieved were gelling activity and emulsification activity (**Table 9**). If a centrifugation step is included after the lysis, and the buffer citrate treatment is done, cold gelling property was also achieved.

### Example 11. Fractioning for gelling, emulsifying and foaming properties (KZ12F)

Yeast cells were lysed by enzymatical treatment for 4 hours. After that, the cell wall was removed and resuspended in buffer citrate and treated for 20' at 121°C. After that, the sample was directly dried. The technological properties achieved were gelling activity in hot conditions, emulsifying and foaming activity, while nutritional profile was improved with higher protein content (**Table 9**).

**Table 9. Nutritional and technological properties of the different ingredients coming from the same biomass**

| **Parameter** | **Protoype 0 ¹** | **KD12F** | **KZ12F** |
|---|---|---|---|
| Moisture | 3.8 | 2.4 | 2.5 |
| Fat | 4.1 | 4.3 | 3.9 |
| Protein | 38.2 | 36.9 | 36.9 |
| Ash | 8.6 | 12.6 | 3.9 |
| Total carbohydrate | 12.6 | 16.5 | 13.1 |
| Sugar | 0.9 | nd | nd |
| Dietary fiber | 31.6 | 27.5 | 27.6 |
| Solubility | 32% | 40% | 41% |
| Emulsifying | Fluid emulsion. stable after 24h small phase separation at the botton | Thick emulsion, not as thick as KZ, more fluid towards | Thick emulsion, like mayonaisse (gel) |
| Foaming | 5% | 5% | 10% (Stable) |
| Gelling | No gel | Fluid Gel | Fluid Gel |
| WHC | 1.4 | 2.37 | 2.10 |
| OHC | 2.15 | 3.55 | 2.04 |

| | | | |
|---|---|---|---|
| *¹ Thermal Inactivated biomass (no extraction process)* | | | |

## Claims

1. Process for obtaining edible biomass which comprises:
a. Placing by-products of the food industry in suitable conditions for their solubilization and/or resuspension,
b. Fermentation of the by-product solution or suspension in the presence of a Crabtree-negative yeast,
c. Inactivation of the Crabtree-negative yeast to obtain the edible biomass.

2. Process, according to claim 1, wherein the Crabtree-negative yeast is selected from: *Candida utilis, Kluyveromyces marxianus, Debaryomyces hansenii* or *Yarrowia lipolytica.*

3. Process, according to any of the previous claims, **characterized in that** a single edible biomass final product, with uniform quantitative and qualitative components, is obtained, departing from a variety of by-products of the food industry.

4. Process, according to any of the previous claims, wherein the edible biomass comprises at least 30% of protein content, at least 10% of soluble fibre and/or at least 1% of fat content; preferably 30 to 80% of protein content, 10 to 70% of soluble fibre and/or 3 to 60% of fat content.

5. Process, according to any of the previous claims, wherein the by-products are non-animal derived by-products.

6. Process, according to any of the previous claims, wherein the by-products are vegetal derived by-products.

7. Process for obtaining a food product which comprises:
a. Obtaining edible biomass by following the process of claims 1 to 6.
b. Fractionating to obtain the food product.

8. Process for obtaining food product, according to claim 7, wherein the fractionation is carried out by using crabtree-negative or crabtree-positive yeasts to obtain a food product with improved gelling, emulsifying, water and oil absorption capacity.

9. Use of a Crabtree-negative yeast for obtaining edible biomass departing from by-products of the food industry.

10. Use, according to claim 9, of a Crabtree-negative yeast selected from: *Candida utilis, Kluyveromyces marxianus, Debaryomyces hansenii* or *Yarrowia lipolytica* for obtaining edible biomass departing from by-products of the food industry.

11. Edible biomass obtained by the process of any of the claims 1 to 6.

12. Edible biomass, obtained by the process of any of the claims 1 to 6, according to claim 11, which comprises: at least 30% of protein content, at least 10% of soluble fibre and/or at least 1% of fat content; preferably 30 to 80% of protein content, 10 to 70% of soluble fibre and/or 3 to 60% of fat content.

13. Use of the edible biomass of claims 11 or 12 for the manufacture of a food product.

14. Food product obtainable by means of the process of claims 7 or 8.

15. Food product, according to claim 14, comprising the edible biomass of claims 11 or 12.
